Europäisches Patentamt

European Patent Office

Office européen des brevets

(11) Publication number: 0 310 710
A1

# (12) EUROPEAN PATENT APPLICATION

(21) Application number: 87201856.9

(22) Date of filing: 28.09.87

(51) Int. Cl.⁴: A61B 17/34

(43) Date of publication of application:
12.04.89 Bulletin 89/15

(84) Designated Contracting States:
AT BE CH DE ES FR GB GR IT LI LU NL SE

(71) Applicant: Zijlstra, Jan Jacobus
Oberonlaan 35
NL-3318 EM Dordrecht(NL)

(72) Inventor: Zijlstra, Jan Jacobus
Oberonlaan 35
NL-3318 EM Dordrecht(NL)

(74) Representative: Boelsma, Gerben Harm, Ir. et
al
Octrooibureau Polak & Charlouis Laan
Copes van Cattenburch 80
NL-2585 GD Den Haag(NL)

(54) Puncture needle.

(57) Puncture needle, in particular for puncturing
deep lying organs of the human body, comprising an
outer needle section (1) and an inner needle section
(2), the inner needle section (2) being slidable be-
tween a retracted position in which its tip (2a) is
located within that of the outer needle section (1)
and an extended position in which its tip (2a) is
extending beyond that of the outer needle section
(1), the inner needle section (2) having - at least
along the length it is extending in the extended
position - a thickness between 0.35 and 0.45 mm.

EP 0 310 710 A1

FIG. 1

## Puncture needle.

The invention relates to a puncture needle, in particular for puncturing deep lying organs of the human body, comprising an outer needle section and an inner needle section, the inner needle section being slideable between a retracted position in which its tip is located within that of the outer needle section and an extended position in which its tip is located beyond that of the outer needle section. Such a needle is known from FR-A-2.293.907. As an example this patent specification mentions a needle having an overall length of 12 cm, the outer needle section measuring 8 cm of length and the inner needle section - in the extended position - extending 4,5 cm beyond the outer section.

In principle such a needle enables the puncturing of deep lying organs. In view of the way of handling of the needle and of the needle diameters described in the patent specification the use of this well-known needle will involve unacceptable risks for most of the organs. As indicated in the patent specification, the needle is - in a first stage and with the inner section in the fully extended position - inserted into the wall of the organ to be investigated, after which the outer needle section - while being guided by the stationary inner section, is inserted as well into the organ to be investigated.

In the said example the smallest needle diameter i.e. that of the extending portion of the inner needle section, is 0,8 mm. The hole formed in the organ wall by a needle of this size would - for many organs -involve an unacceptable risk of leakage of liquid and complications related therewith. In the final stage of use, when the organ wall is punctured by the outer needle section, said hole is even more than doubled in size.

The invention aims at removing the restrictions of the well-known needle above referred to and improving the needle such that even organs, such as the gall bladder, may be punctured without risk.

According to the invention this aim is achieved in that at least the extending end portion of said inner needle section has a thickness between 0.35 and 0.45 mm.

It has been found in practice that a hole formed in an organ wall by means of such a thin needle, tends to quickly close by contraction and consequently does not lead to a real risk in connection with leakage of organ liquid.

Such a small needle thickness, however, would be too small for handling the needle in a way as inducted in the above mentioned patent specification. Apart from the fact, that puncturing of the organ wall by the outer needle section during the second stage would still result in a too large hole, the first insertion stage - with the inner needle section in its retracted position - could not be carried out in connection with the risk for bending and deflection of the very thin extended portion of the needle.

Consequently the above measure according to the invention also implies a different way of handling of the needle.

The two stages, in which the insertion trajectory through the skin and further tissue can be carried out by means of the puncture needle according to the invention, comprises a first stage, which extends up to a location at short distance from the organ and in which only the outer needle section is active as the inner section is taking its retracted position during this stage. The completion of this stage may be checked by echographic means. In the second stage the outer needle section is kept in place while the inner needle section - forming the proper puncture needle - is moved through the outer section outwardly until its tip has punctured the organ wall. During the second stage the longer and thinner inner needle is supported along the greater part of its length - i.e. substantially along the entire length of the outer needle section -against lateral bending or deflection respectively. The perforation caused by the tip of the inner needle section will be substantially smaller than in case the whole puncturing process would be carried out - as would be comparable with the well-known manner of puncturing - with the outer needle section.

For the outer needle section a thickness between 0,7 and 0,8 mm (21-22 gauge), which is usual for a single puncture needle having a length in the order of 100 mm, can be chosen, in case the inner needle section has a thickness between 0,35 and 0,45 mm along its entire length. By using a composite puncture needle of the invention having a size just referred to the risk connected to puncturing (via the skin and the liver) the gall bladder is reduced to an acceptable level. The cavity of such a thin puncture needle is just wide enough for the injection of e.g. a contrast liquid.

It is to be remarked, that single hollow needles having a small thickness (between 0,35 and 0,45 mm) are known per se and used e.g. as an anesthetic needle in dentistry. This well-known needles however are only a few centimeters long.

In a preferred embodiment a releasable locking means is provided, by means of which the outer and inner needle sections may be locked one relative to the other in a mutual position which corresponds to the retracted position of the inner needle section.

The invention further relates to a single hollow needle having a thickness between 0,35 and 0,45 mm, which is adapted to be slidably housed as a core needle in a sheath needle so as to form, together with the latter, the above composite puncture needle.

According to the invention this needle has a length which is larger than 50 mm.

The invention will be hereinafter further explained by way of example with reference to the accompanying drawing.

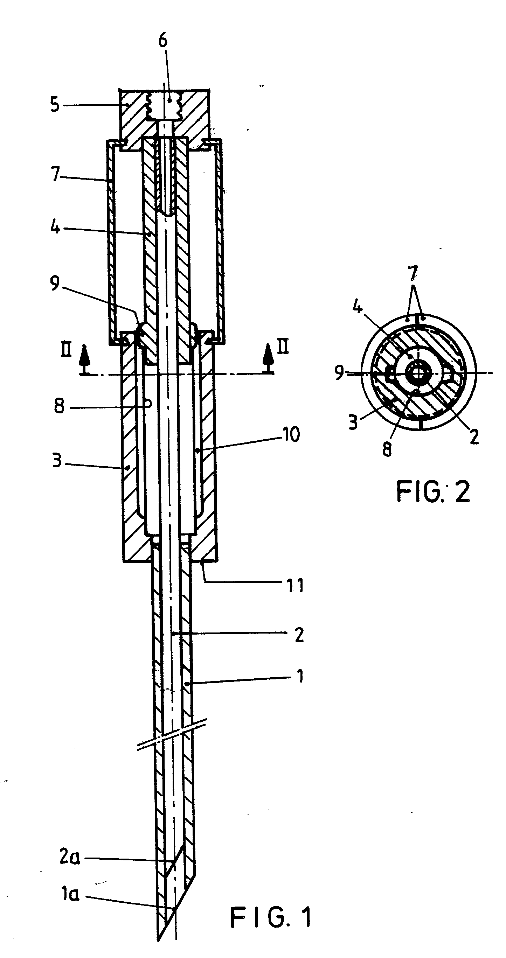

Fig. 1 is a longitudinal sectional view of the puncture needle of the invention, in a condition, in which the inner needle section takes a retracted position relative to the outer needle section and

fig. 2 is a cross-section along the line II-II of Fig. 1.

The puncture needle shown in the drawing, the cross-sections of which have been shown on an enlarged scale, comprises an outer needle section or sheath needle 1 and an inner needle section or core needle 2 slidably housed in the former. The sheath needle has its end 1B faced away from its shapened tip fixed in a hub portion 3. The core needle 2 is, at its end turned away from the beveled tip 2a, strengthened by a bushing 4 and fixed, together with the latter, in a hub portion 5.

The hub portion 5 is provided with a bore for the connection of a hose through which a liquid may be supplied to the hollow core needle 2. In the condition shown in the drawing the core needle is taking a retracted position relative to the sheath needle. In this condition the tip 2a of the core needle 2 lies within the cavity of the sheath needle 1. As shown in the drawing the two needles 1 and 2 are locked together in this mutual position by means of a blocking member 7, which in the example shown is formed by a spacer bushing consisting of two semi-circular shells the inwardly bent terminal edges of which engage in corresponding grooves of the opposed ends of the hub portions 3 and 5. The two shells of the bushing 4 may be simply held together e.g. by adhesive tapes and thus be kept in the operative blocking position.

In the starting condition above described the bushing 4 extends along a certain distance into the rear end of the central bore 8 of the hub portion 3, while guide cams 9 provided on the outer side of the bushing 4 are engaging corresponding guide grooves 10 provided in the wall of the bore 8.

In the condition shown the hub portions 3 and 5 are thus connected to a whole and the puncture needle may be used in the usual manner to carry out the first stage of the inserting trajectory towards the organ to be examined.

As remarked before the arrival at the desired end of the first stage may be determined by echographic means.

Upon completion of the first stage of the insertion the mutual locking of the hub portions 3 and 5 may be simply undone by tearing the adhesive tapes, sothat the shells of the spacer bushing 4 get loose one from the other and may be readily removed. The puncture needle is then prepared for carrying out the second stage of the insertion, in which the hub portion 3 and the sheath needle 1 fixed to it are kept stationary, while the core needle 2 is extended from the sheath needle by exerting axial pressure onto the hub portion 5, until the tip 2a has penetrated into the wall of the organ to be examined. During this stage the hollow needle 2 is guided, for the greater part of its length, by the sheath needle, while the rear end portion of the hollow needle is guidingly supported, via the bushing, by the wall of the bare 8. Due to the cams 9 engaging the longitudinal grooves 10 the core needle 2 is prevented from turning relative to the sheath needle 1.

When the above puncture needle is to be used for carrying out a gall bladder puncture, the sheath needle should have a length (to be measured from the face 11 of the hub portion 3 until the tip 1a) of 10-11 cm, while the core needle should have a length (in entirely extended position and measured from the face 11 of the hub portion 3) of 14-15 cm. For this use a thickness of 0.35 mm of the core needle is sufficiently thin - as indicated above - to avoid complications and is, from a mechanical point of view just enough to enable the core needle to carrying out the second stage of insertion, which is about 4-5 cm long, without the risk of laterial bending or deflection.

The invention is, as will be understood, not limited to the example described above. Within its scope various modifications will be possible.

## Claims

1. Puncture needle, in particular for puncturing deep lying organs of the human body, comprising an outer needle section and an inner needle section, the inner needle section being slideable between a retracted position in which its tip is located within that of the outer needle section and an extended position in which its tip is located beyond that of the outer needle section, characterized in that at least the extending end portion of said inner needle section has a thickness between 0.35 and 0.45 mm.

2. Puncture needle according to claim 1, characterized in that a releasable locking means is provided, by means of which the outer and inner needle sections may be locked one relative to the other in a mutual position which corresponds to the retracted position of the inner needle section.

3. Puncture needle according to claim 1, in which the outer needle section has its end turned away from its sharpened tip fixed in a first hub portion, while the inner needle section extends, through a bore in said first hub portion, into said outer needle section and is fixed to a second hub portion, characterized in that, the two hub portions are locked in a position corresponding to the retracted position of the inner needle section by means of a removable blocking member, which is shaped as a spacer element.

4. Puncture needle according to claims 1-2, characterized in that the end portion of the inner needle section, located adjacent the second hub portion, is strengthened by a guide bushing, which is slidably housed in the bore of said first hub portion.

5. Hollow needle having a thickness between 0,35 and 0,45 mm, which is adapted to be slidably housed as a core needle in a sheath needle so as to form, together with the latter, a puncture needle according to claims 1-3, characterized in that this needle has a length which is larger than 50 mm.

FIG. 2

FIG. 1

European Patent Office

**EUROPEAN SEARCH REPORT**

Application Number

EP 87 20 1856

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 788 119 (BAXTER)<br>* Figures; column 4, lines 10-14 * | 1 | A 61 B 17/34 |
| Y | | 2-5 | |
| Y | FR-A-2 551 977 (TORRE)<br>* Figures; claim 1 * | 2,3 | |
| Y | US-A-3 001 522 (SILVERMAN)<br>* Figures; column 2, lines 7-44 * | 4 | |
| Y | FR-A-2 293 907 (HENRIGUEZ DE GAZTANONDO)<br>* Whole document * | 5 | |
| A | EP-A-0 186 256 (OHTO) | | |

TECHNICAL FIELDS
SEARCHED (Int. Cl.4)

A 61 B
A 61 M

The present search report has been drawn up for all claims

| Place of search | Date of completion of the search | Examiner |
|---|---|---|
| THE HAGUE | 20-05-1988 | STEENBAKKER J. |